(19) Europäisches Patentamt
European Patent Office
Office européen des brevets

(11) Numéro de publication: **0 334 694**
**A1**

# (12) DEMANDE DE BREVET EUROPEEN

(21) Numéro de dépôt: **89400494.4**

(51) Int. Cl.4: **C 12 Q 1/68**

(22) Date de dépôt: **22.02.89**

(30) Priorité: **24.02.88 FR 8802228**

(43) Date de publication de la demande:
**27.09.89 Bulletin 89/39**

(84) Etats contractants désignés:
**AT BE CH DE ES FR GB GR IT LI LU NL SE**

(71) Demandeur: **IRE-CELLTARG S.A.**
**B-6220 Fleurus (BE)**

(72) Inventeur: **Cravador, Alfredo**
**21, avenue des Cygnes**
**1640 Rhode-St.Genese (BE)**

**De Vos-Pierreux, Marie-Joelle**
**6, Tiène à Coulons**
**6520 Feluy (BE)**

**Bollen, Alex**
**65 Gaasbeekstraat**
**1711 Itterbeek (BE)**

(74) Mandataire: **Warcoin, Jacques et al**
**Cabinet Régimbeau 26, avenue Kléber**
**F-75116 Paris (FR)**

Le titre de l'invention a été modifié (Directives relatives à l'examen pratiqué à l'OEB, A-III, 7.3)

(54) **Sonde d'acides nucléiques avec marquage non-radioactif et procédés de préparation.**

(57) La présente invention a pour objet une sonde d'acides nucléiques comportant une séquence d'acides nucléiques, caractérisée en ce que ladite séquence est liée à son extrémité 5', par l'intermédiaire d'un "bras" chimique difonctionnel divalent L, à un "élément de marquage" M, M étant une molécule synthétique ou naturelle, directement ou indirectement détectable de manière non isotopique selon la formule I :

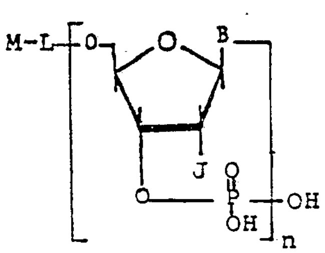

I

dans laquelle
J = H ou OH
n représente le nombre de nucléotides de 1 à 100.000
B = une base d'acides nucléique, purique ou pyrimidique, variable selon les nucléotides, le cas échéant.

EP 0 334 694 A1

## Description

# SONDE D'ACIDES NUCLEIQUES COMPORTANT UN NUCLEOTIDE TERMINAL MODIFIE CHIMIQUEMENT EN 5' (OH) EN VUE DE SON MARQUAGE NON RADIOACTIF ET PROCEDES DE PREPARATION

La présente invention concerne les sondes d'acides nucléiques non radioactives, et des composés nucléotidiques modifiés chimiquement utiles notamment dans la synthèse desdites sondes.

L'emploi de sondes d'acides nucléiques pour détecter et diagnostiquer les maladies génétiques héréditaires, les oncogènes, les maladies virales, bactériennes ou parasitaires, tend à se généraliser et trouve des applications dans les domaines cliniques, vétérinaire et végétal. Ces sondes sont généralement des séquences d'ADN ou d'ARN simples brins capables sous certaines conditions expérimentales de retrouver leurs séquences complémentaires et de s'hybrider avec elles pour former des duplex stables. La méthode de détection classique des hybrides ADN-ADN ou ADN-ARN fait appel à un marquage radioactif ; en général, l'ADN est marqué avec le radioisotope 32P. Lorsque ces sondes sont radioactives, il est alors possible de détecter l'hybridation par comptage ou autoradiographie. L'utilisation des radioisotopes présente cependant de nombreux inconvénients :

a) la perte de l'activité spécifique due à la durée relativement courte de l'isotope ;

b) des problèmes pratiques d'application qui forcent à prendre des mesures de protection contre les radiations et compliquent l'emploi de ces sondes par des non experts ;

c) le prix nécessairement très élevé en raison de ces contraintes.

Récemment, des sondes "froides", car elles ne comprennent pas d'éléments radioactifs, ont donc été développées. Elles mettent en oeuvre des techniques de détection utilisant principalement des systèmes enzyma tiques, par exemple la phosphatase alcaline ou la peroxydase. Ces enzymes réagissent avec un substrat chromogénique incolore et soluble qui donne un produit coloré précipité, permettant ainsi une visualisation rapide de l'hybridation.

De nombreux autres systèmes peuvent être mis en oeuvre qui sont rappelés notamment dans les demandes de brevet EP 63 879 et EP 143 059 auxquelles on pourra se référer pour la meilleure intelligence de la présente demande de brevet. Par exemple, la détection de la sonde hybridée peut se faire par des techniques de fluorescence à l'aide de la fluorescéine.

Les techniques qui ont été développées peuvent se ranger en deux grandes catégories :

1) La première consiste à coupler la sonde à un élément directement détectable. On obtient une détection directe de l'hybride, par exemple par couplage covalent d'une enzyme à la sonde. Ainsi, dans la demande EP 120 376, le polyéthylèneimine a été utilisé pour coupler la peroxydase à l'ADN en présence de glutaraldéhyde. Ou encore, la phosphatase alcaline a été fixée sur le carbone 5 de la thymine via un bras fonctionnel aminé par une liaison amide avec une fonction carboxylique de l'enzyme.

2) La seconde catégorie consiste dans la détection indirecte de l'hybride nucléique. On a recours à des substances intermédiaires qui reconnaissent des motifs fixés sur la sonde. Il s'agit principalement de systèmes basés sur l'interaction entre une sonde biotinylée et l'avidine, l'avidine étant conjuguée le plus souvent à une enzyme. Ce système est fondé sur la grande affinité entre la biotine et l'avidine. On utilise actuellement de préférence la streptavidine à la place de l'avidine. Le système streptavidine-biotine actuellement proposé nécessite cependant d'incorporer des nucléotides modifiés liés à une biotine, dans la sonde nucléique, au moyen d'une nick-translation, technique dite aussi de déplacement de coupure. Cette nick-translation est une technique relativement complexe dans laquelle on a recours à l'emploi d'enzymes telles que la polymérase I de E. Coli et la désoxyribonucléase I diluée.

Les techniques de détection indirecte proposées peuvent également inclure l'utilisation d'anticorps. La sonde peut être porteuse de groupes hapténiques liés à de premiers anticorps antihaptènes qui sont eux-mêmes détectés par d'autres anticorps dirigés contre les premiers, ces autres anticorps étant marqués en application d'une technique non radioactive.

L'inconvénient majeur des techniques proposées, qu'elles relèvent d'une détection directe ou indirecte, réside dans la modification chimique de nucléotides de la sonde qu'elles impliquent.

Des oligodésoxyribonucléotides portant des groupes chimiques permettant le couplage avec des réactifs variés ont été décrits dans la littérature. L'introduction de ces groupes se fait en général sur une ou plusieurs de ses bases, le long de la chaîne oligonucléotidique.

Des modifications chimiques dans les bases ont le désavantage d'interférer avec les appariements des bases lors de la mise en hybridation de l'oligonucléotide avec des séquences homologues.

En outre et surtout, aucune des techniques de détection directe ou indirecte proposées jusqu'à maintenant ne propose de sondes marquées qui soient susceptibles d'être synthétisées entièrement, c'est-à-dire en incluant la fixation de l'élément de marquage à détection directe ou indirecte, dans des conditions classiques de synthèses d'acides nucléiques manuelles ou automatiques, notamment sur support solide.

Le but de la présente invention est donc d'obtenir des sondes d'acides nucléiques :

. offrant une bonne hybridation avec les séquences cibles complémentaires,

. permettant une détection directe ou indirecte par des méthodes non radioactives et avec un seuil de détection aussi bas que possible, et qui, en outre, soient

. facilement synthétisables, c'est-à-dire appropriées à une synthèse d'acides nucléiques automatique ou manuelle, notamment sur support solide.

Pour ce faire, la présente invention consiste à préparer des molécules mixtes composées d'une partie oligonucléotidique ou oligodésoxynucléotidique, et d'une autre partie consistant en un groupement moléculaire possédant des propriétés permettant la détection facile et rapide de cibles d'acides nucléiques complémentaires par des méthodes non radioactives. La partie nucléotidique qui consiste en une séquence d'acides nucléiques définie et homologue d'un fragment complémentaire cible apporte l'énergie de stabilisation et assure l'hybridation avec la molécule d'ADN ou d'ARN que l'on veut détecter. La partie qui fournit la réactivité permettant la détection de l'hybride, directe ou indirecte, encore appelée l'"élément de marquage", est couplée en une extrémité 5′ de la séquence d'acides nucléiques visée précédemment. Ce couplage se fait par l'intermédiaire d'un lien chimique covalent, encore appelé "bras" qui est destiné à éviter un encombrement stérique entre l'élément de marquage et la séquence d'acides nucléiques de la sonde d'une part, et une perturbation éventuelle de l'hybridation de la sonde avec la séquence cible d'autre part.

La présente invention a en effet pour objet une sonde d'acides nucléiques comportant une séquence acides nucléiques (S), ADN ou ARN, caractérisée en ce que ladite séquence S est liée à son extrémité 5′, par l'intermédiaire d'un bras chimique divalent (L), avec un élément de marquage (M), M étant une molécule, synthétique ou naturelle, directement ou indirectement détectable, de manière non isotopique, selon la formule S-L-M.

La modification chimique du nucléotide terminal de la séquence d'acides nucléiques pour sa fixation à l'élément de marquage se fait en 5′, de telle sorte que l'on peut détailler la formule I de la manière suivante. Si S représente une séquence de n acides nucléiques

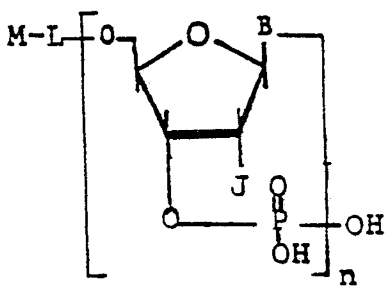

I

dans laquelle
- J = H ou OH
- n représente le nombre de nucléotides de 1 à 100.000
- B est une base d'acides nucléique, purique ou pyrimidique,variable selon les nucléotides.

On rappelle que les acides nucléiques sont des polymères de nucléotides ; ribonucléotides dans le cas des RNA, désoxyribonucléotides dans le cas des DNA. Les monomères, c'est-à-dire les nucléotides, sont, rappelons-le, constitués dans le cas du RNA par de l'acide phosphorique, un ose à 5 atomes de carbone, le ribose (J = OH) et l'une des quatre bases fondamentales : adénine, guanine, cytosine, uridine. On rencontre plus rarement des bases ou nucléosides dits mineurs, tels les bases méthylées ou hydroxylées, le dihydro-uracile et la pseudo-uridine. Dans le cas des DNA, l'ose des désoxyribonucléotides est le D-2-désoxyribose (J = H) et les quatre bases principales sont l'adénine, la guanine, la cytosine et la thymine ; dans de rares cas, la cytosine est remplacée par la méthylcytosine ou l'hydroxyméthylcytosine. Une des caractéristiques essentielles des polynucléotides est la liaison inter-nucléotidique 3′-5′ phosphodiester.

La synthèse du composé I peut donc se réaliser par synthèse inter-nucléotidique classique avec élongation à l'extrémité 5′ du nucléotide terminal. Pour ce faire, le lien chimique covalent entre le bras et la sé quence d'acides nucléiques en 5′ doit être stable, notamment dans les conditions de clivage de l'oligonucléotide ou oligodésoxynucléotide du support solide, le cas échéant, et dans les conditions de déprotection sur les bases et les phosphates qui interviennent dans les synthèses inter-nucléotidiques.

C'est pourquoi dans un mode préférentiel de réalisation selon la présente invention, le bras chimique consiste en un reste chimique difonctionnel divalent pouvant créer un lien carbamate avec le groupe hydroxyl-5′ de la séquence d'acides nucléiques , reste chimique notamment de formule II:

$-L'-\overset{O}{\overset{\|}{C}}-$ où -L′- est un reste aminé à ses deux extrémités.

Plus particulièrement, L représente le reste

$-NH-Alk-NH-\overset{O}{\overset{\|}{C}}-$, dans lequel Alk représente une chaîne alkyle droite ou ramifiée de 2 à 20 atomes de carbone. Le bras L de formule II réalise un lien carbamate stable, dans les conditions de clivage et de déprotection visées précédemment, avec l'extrémité 5′(OH) de la séquence d'acides nucléiques.

Lorsque L a pour formule III =

$-NH-Alk-NH-\overset{O}{\overset{\|}{C}}-$ , Alk représente de préférence une chaîne droite de 2 à 12 carbones.

De préférence encore, Alk représente le groupe $-(CH_2)_6-$.

L'élément de marquage M peut représenter une molécule directement détectable classique. Notamment M peut être choisie parmi les enzymes appropriées à la révélation par un substrat chromogénique parmi lesquelles on peut citer particulièrement la microperoxydase et la phosphatase alcaline.

Selon la présente invention, M peut toutefois avantageusement représenter une macromolécule sur laquelle sont fixés plusieurs groupements directement détectables comme des enzymes ou des molécules fluorescentes, telles que la fluorescéine. Dans ce cas, M peut représenter un polymère synthétique comme le polyéthylèneimine sur lequel sont fixés de tels groupements comme des enzymes ou des molécules fluorescentes.

On peut citer plus particulièrement, selon la présente invention, le polyfluorescéinyle-polyéthylèneimine. Ce type de composé se traduit par une amplification du marquage et donc une baisse du seuil de détection.

M peut également représenter une molécule indirectement détectable classique, comme la biotine ou un de ses dérivés portant une chaîne hydrocarbonée par laquelle se fait le lien avec L et contribuant à augmenter la distance avec l'oligonucléotide ou encore une macromolécule telle qu'un anticorps. Selon la présente invention, M peut avantageusement représenter une macromolécule sur laquelle sont fixés plusieurs groupements indirectement détectables, par exemple un polymère synthétique polybiotinylé comme le polybiotinyl-polyéthylèneimine.

La présente invention a donc aussi pour objet à titre de produit intermédiaire utile notamment comme élément de marquage d'une sonde d'acides nucléiques un polymère synthétique, notamment polyamine comme le polyéthylèneimine sur lequel sont fixés plusieurs groupement directement détectables comme des molécules fluorescentes, notamment le polyfluorescéinylpolyéthylèneimine comme moyen de détection directe ou le polybiotinylpolyéthylèneimine comme moyen de détection indirecte.

La présente invention a de même pour objet un procédé de préparation d'une sonde de formule I comportant :

a) la synthèse d'une séquence d'acides nucléiques de formule Ia :

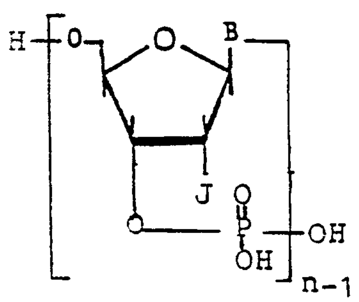

Ia

notamment par toute méthode de synthèses d'assemblage inter-nucléotidique manuelle ou automatique connue, de préférence sur support solide, caractérisé en ce que ladite séquence est soumise, de préférence par une même méthode de synthèse, notamment sur support solide, à

b) une extension en son extrémité 5'(OH) libre, sur l'extrémité 3'(OH) d'un synthon nucléotidique ou désoxynucléotidique, ce dernier étant protégé en 3' par un groupe phosphorylé approprié au type de synthèse employé et, étant porteur en 5' d'un bras chimique L protégé à son extrémité libre par un groupe $R_1$ de formule IV

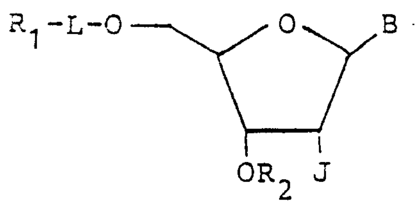

IV

dans laquelle

- J, B, L, $R_1$ ont les significations données précédemment, B étant éventuellement protégée,
- $R_2$ représente H ou tout groupe phosphorylé, éventuellement protégé, approprié à l'introduction du composé de formule IV à l'extrémité 5' d'un autre nucléotide pour un type donné de synthèse d'assemblage inter-nucléotidique, pour donner le composé de formule Ib

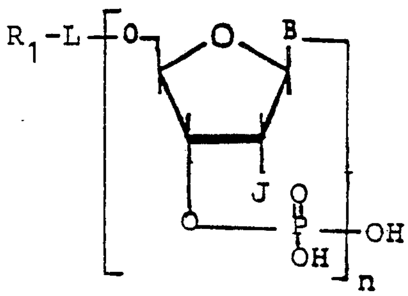

Ib

le groupe $R_1$, après activation, notamment par l'intermédiaire d'un groupement difonctionnel, tel qu'un diépoxyde, établit

c) le couplage avec la molécule, "élément de marquage" M.

Lorsque l'"élément de marquage" M consiste en un polymère chimique biotinylé ou fluorescéinylé, ledit polymère chimique est marqué dans des conditions de non saturation de telle sorte qu'il lui reste des groupes nucléophiles pour que le polymère marqué se couple à l'oligonucléotide porteur du "bras" chimique.

Lorsque la synthèse de la sonde de formule I est entièrement réalisée sur support solide, la réaction de couplage de l'étape c) peut être effectuée quand le substrat, produit des étapes a) et b), est encore fixé sur le support solide, ou bien après clivage, quand la stabilité de la liaison avec la molécule M est insuffisante dans les conditions de clivage.

Lorsque M représente une macromolécule sur laquelle sont fixés un ou plusieurs groupements directement ou indirectement détectables, la fixation desdits groupements sur la macromolécule peut avoir lieu avant ou après le couplage de cette dernière sur le substrat, produit des étapes a) et b).

L'activation pour le couplage de l'étape c) peut avoir lieu avec un diépoxyde, comme on l'a vu, mais également avec le diisocyanatohexane ou le diester N-hydroxysuccinimidique de l'acide subérique.

A titre de produit intermédiaire, utile notamment pour la synthèse d'une sonde de formule I, selon le procédé précédent, la présente invention a également pour objet un composé synthétique nucléotidique, encore appelé synthon, caractérisé en ce qu'il est représenté par la formule IV :

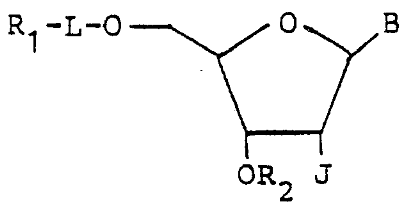

IV

dans laquelle :

J = H ou OH

B représente une base d'acides nucléiques éventuellement protégée

L est un bras chimique difonctionnel divalent

$R_1$ est un groupe protecteur de la fonction terminale libre de L

$R_2$ représente H ou tout groupe phosphorylé, éventuellement protégé, approprié à l'introduction du synthon à l'extrémité 5′ d'un autre nucléotide pour un type donné de synthèse d'assemblage inter-nucléotidique.

$R_2$ peut représenter à titre illustratif en cas de synthèse au phosphoramidite sur support solide le groupe cyanoéthyldiisopropylphosphoramidite de formule V

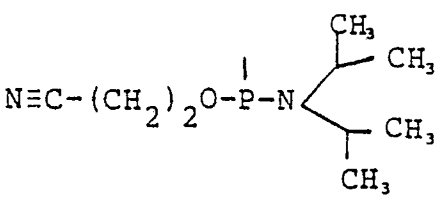

V

Cette phosphorylation en 3′ (OH) n'est évidemment pas limitative, on peut notamment envisager la synthèse d'un phosphate triester ou diester dans le cas de synthèse au phosphotri- ou diester.

De préférence, le bras chimique difonctionnel L peut être représenté par un reste de formule $L'-\underset{\underset{O}{\|}}{C}-$ dans lequel L′ est un reste diaminé.

Lorsque L représente le reste, $L'-\underset{\underset{O}{\|}}{C}-$

où L′ est un reste diaminé, par exemple lorsque L a pour formule $-HN-(CH_2)_6-NH-\overset{O}{\overset{\|}{C}}-$, on peut citer comme groupe protecteur $R_1$ approprié de fonction amine terminale, toujours à titre illustratif, le groupe diphénylisopropyloxycarbonyle, labile en conditions acides, de formule VI :

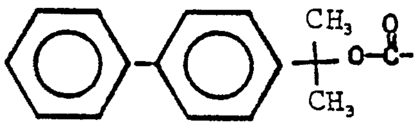

VI

Un produit conforme à l'invention répond à la formule VII

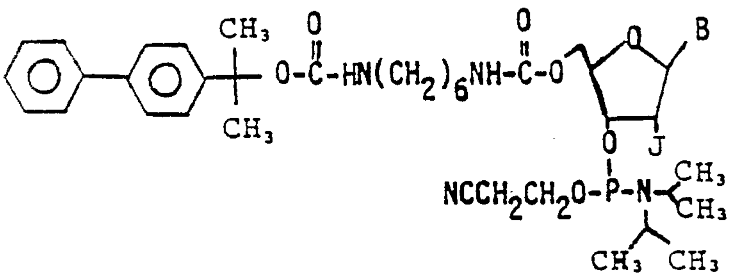

VII

dans laquelle B et J ont les significations données précédemment.

Il convient de souligner que les composés de formule IV sont appliqués à titre illustratif au marquage d'une sonde d'acides nucléiques et que d'autres applications peuvent donc en être faites.

On peut envisager par exemple de fixer une séquence d'acides nucléiques sur un support solide à l'aide de tels composés.

La présente invention a en outre pour objet un procédé de préparation d'un composé de formule IV comportant les étapes suivantes :

1) la protection sélective du groupe hydroxylique en 3′ du nucléoside de formule VIII

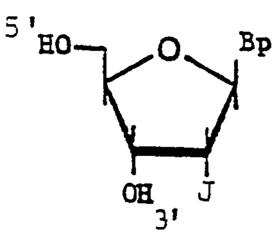

VIII

dans laquelle Bp est une base d'acides nucléiques protégée et J = H ou OH.

2) L'activation sélective du groupe hydroxylique en 5′.

3) Une réaction de couplage en 5′ pour donner le "bras" chimique monoprotégé de formule $R_1$ -L où L a la signification donnée précédemment.

4) La déprotection de la fonction hydroxylique en 3' et éventuellement

5) la phosphorylation avec un réactif qui permet le couplage en phase solide du nucléotide ainsi modifié en 3′, couplage à l'extrémité 5′ d'un autre nucléotide par un type donné de synthèse d'assemblage internucléotidique.

Lorsque le bras L peut être représenté par la formule $L'-\underset{O}{\underset{\|}{C}}-$ dans laquelle L′ représente un reste diaminé, l'activation sélective du 5′OH se fera de préférence avec le groupe carbonyle diimidazole (CDI). La réaction de couplage 3) ci-dessus se fera avec le reste diaminé monoprotégé sur une amine terminale de formule $R_1$-L′. $R_1$ représentant de préférence le groupe diphénylisopropyloxycarbonyle.

La protection sélective du groupe hydroxylique en 3′ se fera de préférence avec le groupe diméthyltertiobutylsilyle, ce groupe silylé étant stable lors de la réaction d'activation 2 avec le CDI. Par ailleurs, ce groupe convient bien d'une manière générale pour la suite du chemin de synthèse.

Lorsque la protection sélective de l'étape 1) du groupe 3′OH se fait avec le groupe silylé mentionné précédemment, la déprotection de l'étape 4) peut se faire avec le fluorure de tétrabutylammonium.

Dans l'étape 5), la phosphorilation en cas de synthèse au phosphoramidite pourra se faire avec le di isopropylaminocyanoéthoxychlorophosphine de formule IX

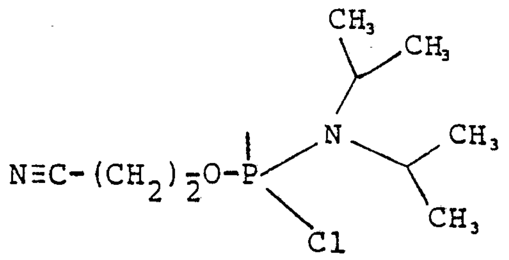

IX

L'étape 1 du précédent procédé peut également se détailler de la manière suivante :

1a) - Protection sélective de l'extrémité 5'OH du nucléoside de formule VIII, de préférence cette protection se faisant avec le diméthoxytrityle (DMT) labile à pH acide.

1b) - Protection du 3'OH du composé de formule VIII par un groupe labile dans des conditions douces et neutres, de préférence, cette protection se faisant par le groupe diméthyltertiobutylsilyle. Ce groupe étant stable lors de la réaction de déprotection 1c) et d'activation 3).

1c) - Déprotection sélective du DMT pour libérer le 5'OH.

D'autres caractéristiques et avantages de la présente invention apparaîtront à la lumière des exemples qui vont suivre. L'exemple IV fait référence à la figure 1 sur laquelle est représentée la visualisation de l'hybridation d'une sonde selon l'invention de l'ADN du virus $HPV_{16}$.

EXEMPLE I : Préparation du dérivé 5'[2-(4-biphénylyl)-propyl-2-oxycarbonylamido-6-hexylamidocarboxy]thymidine-3'-0-(2-cyanoéthyl-N,N-diisopropylphosphoramidite)

Le dérivé 5'[2-(4-biphénylyl)-propyl-2-oxycarbonylamido-6-hexylamidocarboxy] thymidine-3'-0-(2-cyanoéthyl N,N-diisopropylphosphoramidite) répond à la formule :

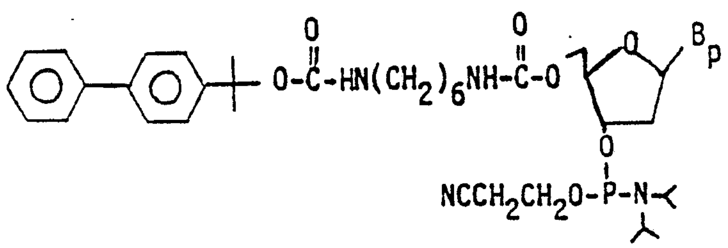

dans laquelle $B_p$ représente la base thymine protégée.

L'approche suivie a comme objectif la préparation d'un synthon nucléotidique portant d'une part, les groupes chimiques qui permettent son introduction dans un oligodésoxyribonucléotide au cours du dernier cycle d'élongation dans les conditions de synthèse automatique habituelles, et d'autre part, la chaîne carboxylalkylaminée nécessaire au couplage de la molécule d'ADN avec un"élément de marquage"M. Ce synthon a l'avantage de fournir à l'utilisateur un outil permettant la synthèse d'une molécule d'ADN couplable, sans sortir de la routine de synthèse automatique ou manuelle.

Le groupe cyanoéthyldiisopropylphosphoramidite est approprié à une synthèse au phosphoramidite.

Un lien carbamate lie le groupe 5'OH du nucléotide à l'hexanediamine dont l'extrémité aminée est protégée par le diphénylisopropyloxycarbonyle labile en conditions acides.

Ce synthon nucléotidique est protégé et muni de fonctions réactives appropriées permettant leur introduction en phase solide à l'extrémité 5' de n'importe quel oligodésoxynucléotide synthétique dans la mesure où le lien carbamate est stable:

1) dans les conditions d'élimination des groupes protecteurs présents sur les bases et les phosphates, et

2) dans les conditions de clivage de l'oligodésoxynucléotide du support solide.

Un premier chemin réactionnel est présenté dans le schéma 1 ci-après qui comporte les étapes suivantes :

I) protection sélective du 5'-OH par le dimétoxytrityle (DMT) labile à pH acide

II) protection du 3'-OH par le groupe lévulinique labile en présence d'hydrazine

III) déprotection sélective du DMT pour libérer le 5'-OH

IV) activation du 5'-OH avec le carbonyldiimidazole

V) réaction avec l'hexylènediamine fournissant un lien carbamate stable et un groupe aminé à une extrémité

VI) protection de l'amine avec le diphénylisopropyloxycarbonyle labile en présence d'acide

VII) déprotection sélective du groupe lévulinique à l'hydrazine pour libérer le 3'-OH

VIII) phosphorylation en 3'-OH. L'exemple du phosphoramidite montré dans le schéma n'est pas limitatif ; on peut envisager aussi la synthèse d'un phosphate triester ou diester ou d'un phosphonate.

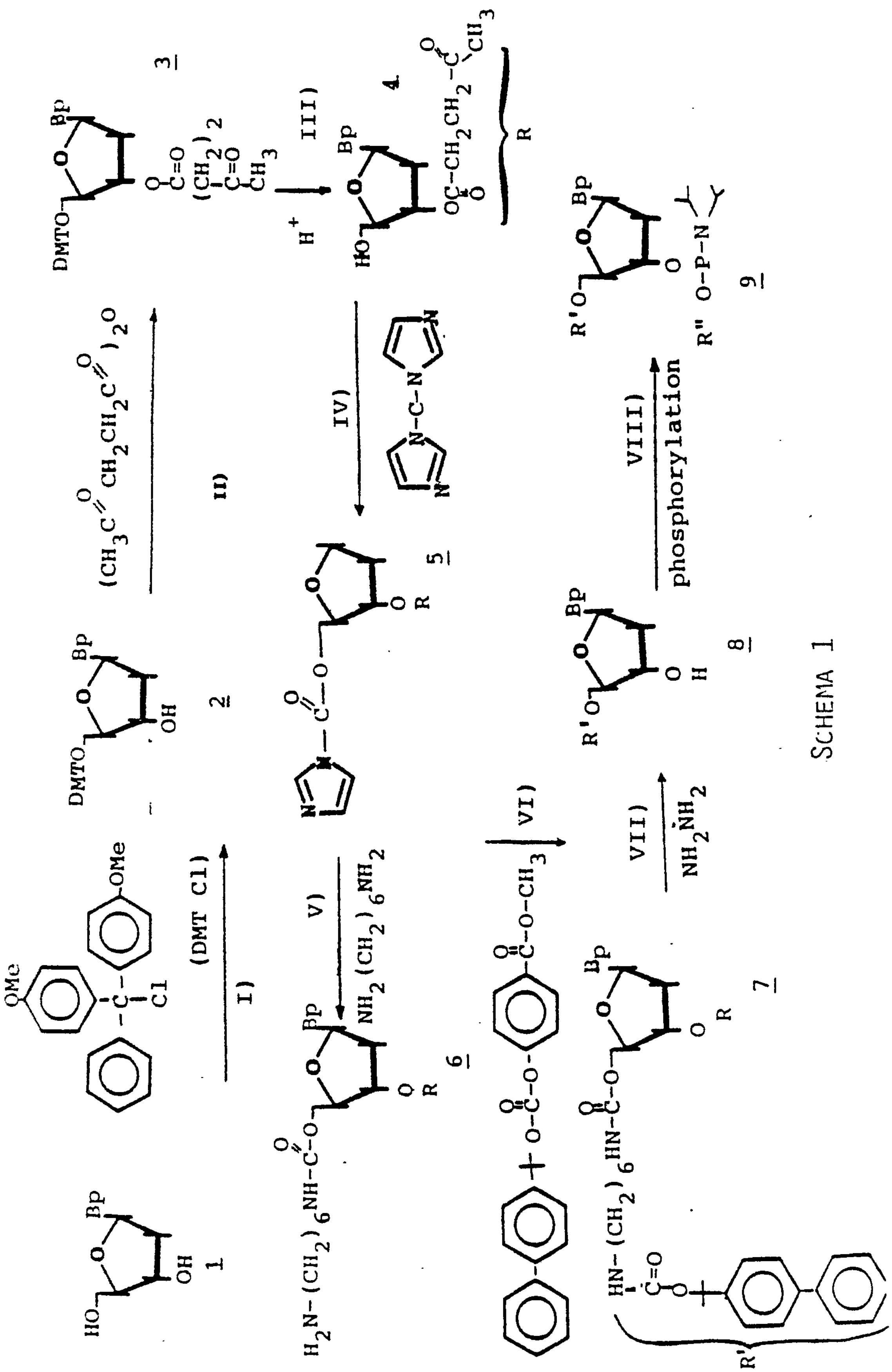


1 2 3 4 5 6 7 8 9

SCHEMA 1

Le chemin réactionnel présenté dans le schéma 1 est avantageusement modifié dans le schéma 2 suivant en raison des problèmes posés par le groupe lévulinique lors de l'activation du groupe hydroxylique en 5′ avec le carbonyldiimidazole (CDI). Bien que parfaitement stable dans les conditions réactionnelles utilisées, le dérivé 4 (schéma 1) exige un excès d'au moins deux fois de CDI. Comme le produit activé 5 (schéma 1) n'est pas isolable, l'excès de CDI consomme inutilement un équivalent molaire du dérivé aminé et complique la purification.

Dans le chemin réactionnel présenté dans le schéma 2 ci-après, le groupe lévulinique a été remplacé par le groupe diméthyltertiobutylsilyle qui peut aussi être retiré dans des conditions douces et neutres et reproduit avec succès les conditions réactionnelles de la substitution avec le 3′-0-diméthyltertiobutylsilyl-5′-0-carbonylimidazolthymidine (12 ; schéma 2), préparé à partir du dérivé 11 (schéma 2) par réaction de son groupe 5′OH avec le carbonyldiimidazole.

Le groupe silylé ne gène pas la réaction d'activation avec le CDI et convient bien pour la suite du chemin synthétique qui a été réalisé comme décrit dans le schéma 2.

La réaction de substitution avec la 1,6-diaminohexane est relativement complexe à cause du caractère bifonctionnel du dérivé nucléophilique. Par contre le 1-aminohexane donne dans les mêmes conditions une réaction nette. Pour simplifier cette réaction de substitution, nous avons étudié donc la réactivité de l'hexane diamine vis-à-vis du 2-(4-biphénylyl)prop-2-yl-4-méthoxycarbonylphényle carbonate (10, schéma 2) dans le but d'obtenir la protection d'un seul groupe aminé. Le dérivé monoprotégé 13 (schéma 2) a pu être isolé avec un rendement supérieur à 90 %. Ce réactif monofonctionnel réagit efficacement avec le 3′-0-silylé-5′-0-carbonylimidazolthymidine.

Le dérivé 14 (schéma 2) entièrement protégé, a donc été obtenu par réaction de l'hexanediamine monoprotégée 13 (schéma 2) avec le composé 12 (schéma 2) activé en 5′ par le groupe carbonylimidazole. La déprotection de 14 avec le fluorure de tétrabutylammonium permet de libérer le groupe hydroxylique en 3′ et d'accéder au dérivé 8 (schéma 2). La phosphorylation de 8 avec le diisopropylaminocyanoéthoxychlorophosphine fournit le phosphoramidite 9. Toutes les étapes ont été réalisées avec d'excellents rendements. Le dérivé (9) a été caractérisé par RMN du $^{1}H$ et du $^{31}P$.

SCHEMA 2

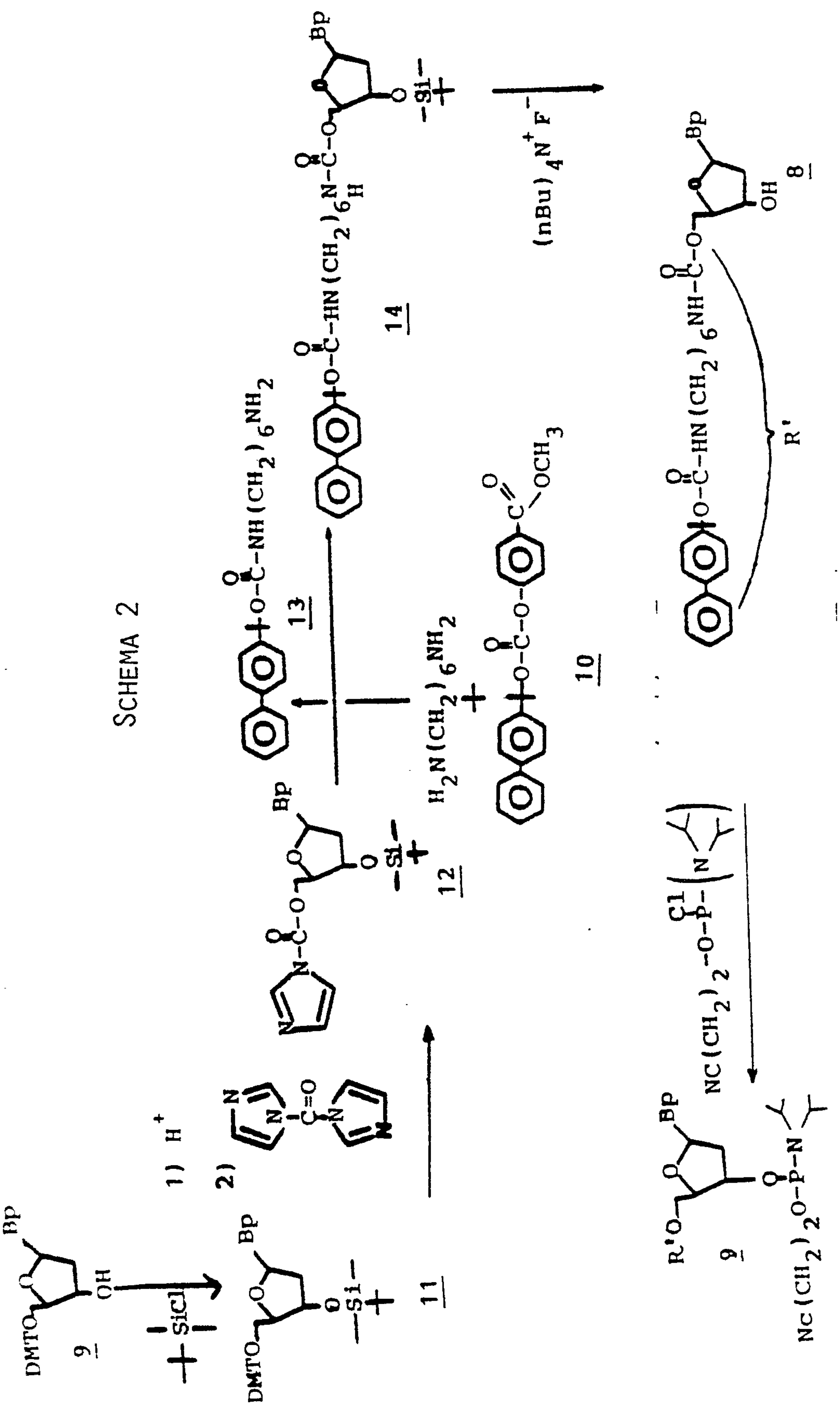

Le dérivé de départ de la synthèse, la thymidine, a été transformé en 3′-tertbutyldiméthylsilylthymidine 11 en trois étapes essentiellement, comme décrit ci-dessous.

Synthèse de la 3′-tertiobutyldiméthylsilylthymidine à partir de la thymidine

1ère étape.

La thymidine (1,25 g $5.10^{-3}$ mole) est mise en solution dans la pyridine anhydre (25 ml). Le chlorure de 4,4′-diméthoxytrityle 1,99 g ($6.10^{-3}$ mole) est additionné et le mélange réactionnel est ensuite agité pendant 2 h à 20° C. Du méthanol (10 ml) est additionné et, après 10 minutes d'agitation, la solution est diluée avec 50 ml de dichlorométhane et lavée avec 3 x 50 ml d'une solution de $NaHCO_3$ 5 % dans l'eau. La solution est séchée sur $Na_2SO_4$, filtrée et les solvants évaporés. Le résidu est purifié sur colonne de silice Merck 9835 (60 g) en éluant d'abord avec du dichlorométhane (300 ml) : pyridine (0,01 %), ensuite avec du dichlorométhane (150 ml) : MeoH (1 %) : pyridine (0,01 %), et finalement avec 450 ml de dichlorométhane : MeoH (2 %) : pyridine (0,01 %). Les fractions contenant la 5′-0-(4,4′-diméthoxytrityl)thymidine sont concentrées. Le résidu est repris dans du dichlorométhane (40 ml) et précipité goutte à goutte sous agitation dans l'hexane (350 ml). Le précipité blanc est séché sous vide. Poids obtenu 2,18 g, Rendement = 90 %.

2ème étape.

De la 5′-0-(4,4′-diméthoxytrityl)thymidine (4,35 g, $8.10^{-3}$mole) et de l'imidazole (2,25 g, 3,35 $10^{-2}$ mole) sont introduits dans un ballon bicol sous argon et additionnés d'une solution de diméthyltertiobu tylchlorosilane (2,4 g, 1,6 $10^{-2}$ mole) dans le diméthylformamide (40 ml). Le mélange réactionnel est agité 5 h à 20° C et ensuite versé dans un mélange d'eau glacé (450 ml). Le précipité est filtré et repris dans du dichlorométhane (100 ml). La phase organique est lavée une fois avec une solution aqueuse de $NaHCO_3$ 5 % (100 ml), séchée sur $MgSO_4$ et concentrée. Le résidu est séché sous vide. Poids obtenu 5,13 g, Rendement = 97 %.

3ème étape.

A 15 g (2,28 $10^{-2}$ mole) de 5′-0-(4,4′-diméthoxytrityl)-3′-0-diméthyltertiobutylsilylthymidine est additionnée une solution d'acide trichloroacétique 3 % dans du dichlorométhane (300 ml). Le mélange réactionnel est agité 1/2 h à 20° C, ensuite lavé jusqu'à neutralisation avec une solution aqueuse de $NaHCO_3$ 5 %, séchée sur $Na_2SO_4$, filtrée et concentrée par évaporation des solvants. Le résidu est purifié sur colonne de silice Merck 9835 (350 g) en éluant d'abord avec du dichlorométhane pur, ensuite avec du dichlorométhane-acétate d'éthyle (70:30, V:V) et enfin avec de l'acétate d'éthyle dichlorométhane (50:50, V:V). Les fractions contenant le produit sont évaporées. Le résidu solide blanc est séché sous vide. Poids obtenu 8,7 g, Rendement = 89 %. Le produit a été contrôlé par CCM de silice et caractérisé par RMN du $^1H$.

Procédé de préparation du 2-(4-biphénylyl)-propyl-2-oxycarbonylamido-6-hexylamine (13)

La 1,6-diaminohexane (2,034 g, 1,75 $10^{-2}$ mole) et le 2-(4-biphénylyl)-prop-2-yl-4-méthoxycarbonylphénylcarbonate (10) (1,71 g, 4,38 $10^{-3}$ mole) sont mis en solution dans le dioxanne (60 ml). Le mélange réactionnel est agité à 21° C pendant 26 h. Du $Na_2CO_3$ 5 % dans l'eau est additionné au mélange. La phase aqueuse est extraite avec 100 ml de dichlorométhane. La phase organique est lavée une fois avec du $Na_2CO_3$ 5 % dans l'eau et deux fois avec de l'eau, puis séchée sur sulfate de sodium et filtrée. Les solvants sont évaporés. Le résidu est purifié par chromatographie sur colonne de silice Merck 9835 en éluant d'abord avec du dichlorométhane (600 ml), ensuite avec du dichlorométhane 98 : méthanol 2 (200 ml) et finalement avec du dichlorométhane 93 : méthanol 5 : $Et_3N$ 2 (100 ml). Les fractions contenant le produit sont évaporées et le résidu séché sous vide. Poids obtenu : 1,08 g ; Rendement = 90 %. Le produit a été contrôlé par CCM de silice et caractérisé par RMN du $^1H$.

Ce type de dérivé est extrêmement utile car il possède deux fonctions nucléophiles parfaitement équivalentes, dont une est sélectivement et temporairement protégée. Le groupe aminé libre peut réagir pour former un lien covalent avec une molécule possédant un centre électrophile. Par la suite, le groupe protecteur de la deuxième fonction aminée peut être éliminé dans des conditions acides très douces régénérant un nouveau centre nucléophile.

Procédé de préparation de la 5′-[2-(4-biphénylyl)-propyl-2-oxycarbonylamido-6-hexylamidocarboxy-3′-tertiobutyldiméthylsilyl-thymidine 14

A une solution de 3′-tertbutyldiméthylsilylthymidine (1,068 g, 3 $10^{-3}$ mole) dans le dioxanne (12 ml), placée dans un bicol sous argon, est ajoutée une solution de carbonyldiimidazole (0,534 mg, 3,3 $10^{-3}$ mole) dans le dioxanne (4,8 ml). Le mélange réactionnel est agité 5 h à 50°C et ensuite additionné d'une solution de 2-(4-(biphénylyl)-propyl-2-oxycarbonylamido-6-hexylamine 13 (1,41 g, 4 $10^{-3}$ mole) dans le diméthylformamide (7,2 ml). Le mélange réactionnel est agité 20 h à 20° C et est ensuite hydrolysé avec une solution aqueuse de $NaHCO_3$ 5 % (100 ml). La phase aqueuse est extraite avec du dichlorométhane (100 ml) et la phase organique est lavée deux fois avec une solution aqueuse de $NaHCO_3$ 5 %, filtrée et séchée sur $Na_2SO_4$. Les solvants sont évaporés et le résidu purifié sur colonne de silice Merck 9835 en éluant avec du dichlorométhane. Les fractions contenant le produit sont évaporées. Poids obtenu : 2,06 g ; Rendement = 93 %. Le produit a été contrôlé par CCM de silice et caractérisé par RMN du $^1H$.

Procédé de préparation du 5′-[2-(4-biphénylyl)-propyl-2-oxycarbonylamido-6-hexylamidocarboxy]thymidine 8

Du fluorure de tétrabutylammonium (3,6 g, 11,2 $10^{-3}$ mole) est additionné à une solution de 5′-[2-(4-biphénylyl)-propyl-2-oxycarbonylamido-6-hexylamidocarboxy]-3′-O-tertbutyldiméthylsilyl-thymidine 14 (2,06 g, 2,8 $10^{-3}$ mole) dans le tétrahydrofuranne. Le mélange réactionnel est agité 1/2 h à 20°C et ensuite les solvants sont évaporés. Le résidu est purifié par chromatographie sur colonne de silice Merck 9835 en éluant d'abord avec du dichlorométhane, ensuite avec du $CH_2Cl_2$:MeoH (95:5, V:V). Les fractions contenant le produit sont évaporées. Poids obtenu 3,99 g Rendement = 93 %. Ce produit a été contrôlé par CCM de silice et caractérisé par RMN du $^1H$.

Procédé de préparation du 5′-[2-(4-biphénylyl)-propyl-2-oxycarbonylamido-6-hexylamidocarboxy]-3′-0-[(N,N-diisopropylamino)-cyanoéthoxyphosphino]thymidine 9.

A une solution de 5′-[12-(4-biphénylyl)-propyl-2-oxycarbonyl-amido-6-hexylamidocarboxy]thymidine 8 (0,622 g, $10^{-3}$ mole) dans le tétrahydrofuranne anhydre (8µ) agitée sous argon dans un bicol, sont additionnés successivement de la diisopropylamine (0,68 ml) et du cyanoéthoxydiisopropylaminochlorophosphine (0,39 ml, 1,9 $10^{-3}$ mole). Le mélange réactionnel est agité 1/2 h à 20°C et filtré. De l'acétate d'éthyle (10 ml) est additionné au filtrat et la solution obtenue est lavée trois fois avec 10 ml d'une solution aqueuse de $NaHCO_3$ 5 %, séchée sur $Na_2SO_4$, filtrée et ensuite concentrée à sec. Le résidu est purifié par chromatographie sur colonne de silice Merck 9835 en éluant avec de l'acétate d'éthyle:hexane (70:30, V:V). Les fractions contenant le produit sont évaporées, le résidu est dissous dans 4 ml de dichlorométhane et ensuite précipité dans 50 ml d'hexane à -90° C avec agitation et sous argon. Le solide blanc est filtré et séché sous vide. Poids obtenu : 0,68 g ; Rendement = 82 %. Le produit a été contrôlé par CCM de silice et caractérisé par RM du $^1H$ et du $^{31}P$.

EXEMPLE II - Etude de la réactivité du dérivé 9

Ce type de dérivé de nucléotide peut être utilisé pour lier de façon covalente des molécules d'ADN synthétiques à des macromolécules naturelles telles que des enzymes, à des polymères synthétiques ou à des molécules fluorescentes ou pouvant former des complexes très stables avec des macromolécules comme par exemple la biotine. Il trouve des applications dans les recherches biologiques, biomédicales et en particulier dans le domaine des diagnostics. Ce dérivé peut être introduit en une seule étape à l'extrémité d'un oligodésoxyribonucléotide dans des conditions standardisées de synthèse automatique en phase solide, fournissant en un cycle d'élongation une fonction chimique réactive éloignée du squelette oligonucléotidique.

Le phosphoramidite 9 a été mis en réaction en solution dans l'acétonitrile avec le 3′-0-levulinylthymidine en présence de tétrazole, et la cinétique de la réaction de couplage a été suivie en parallèle avec celle, connue, du dérivé 3′-0-[(N,N-diisopropylamino)-cyanoéthoxyphosphino]-5′-0-(diméthoxytrityl)thymidine par chromatographie sur couche mince (CCM) et par RMN du $^{31}P$.

Les résultats montrent que la vitesse de formation du lien internucléotidique est comparable avec les deux dérivés. Le couplage des dérivées de formule générale 9 en phase solide se fait dans des conditions identiques ou proches de celles des dérivées habituellement utilisés en synthèse oligonucléotidique.

La labilité du groupe protecteur de l'amine terminale a également été étudié en solution, en parallèle avec celle du dérivé DMT de la thymidine. Dans les conditions habituellement employées (acide trichloroacétique 3 % dans le $CH_2Cl_2$), les vitesses de déprotection des deux groupes sont comparables. Ces résultats ont été confirmés en phase solide.

Le dérivé 9 (où Bp = T) a été condensé dans l'appareil automatique de synthèse des oligonucléotides avec la thymidine fixée sur le support solide et le groupe protecteur de l'amine a été enlevé automatiquement. Toutes les conditions (temps, concentrations et solvants) habituellement utilisées au cours d'un cycle d'élongation oligonucléotidique ont été reproduites sur un synthétiseur ABS 380A. Après clivage à l'ammoniaque du support solide, le produit brut a été analysé par HPLC et le résultat comparé avec celui obtenu après condensation effectuée en parallèle avec le dérivé 3′-0- [(N,N-diisopropylamino) cyanoéthoxyphosphino]-5′-0-(diméthoxytrityl)thymidine.

On obtient dans chaque cas un produit quasiment pur. Les deux dimères se distinguent nettement par leur temps de rétension en HPLC. Ces résultats montrent que les réactions de condensation, d'oxydation et de déprotection en phase solide sont efficaces et ne dégradent pas le nouveau dimère. En outre, une étude effectuée sur la stabilité de la nouvelle liaison carbamate confirme qu'elle est résistante au traitement ammoniacal dans les conditions de déprotection des bases.

Couplage en phase solide du dérivé 9 avec un (oligo)nucléotide fixé sur un support insoluble

Le dérivé 9 solubilisé dans l'acétonitrile à une concentration de 0,1 M est activé par le tétrazole 0,5 M dans le même solvant et condensé avec le groupe hydroxylique en position 5′ de la thymidine fixée sur un polymère insoluble habituellement utilisé en synthèse oligodésoxyribonucléotidique sur un synthétiseur ABS 380A. Toutes les conditions sont identiques à celles utilisées dans un cycle d'élongation oligonucléotidique (N.D. Sinha, J. Biernat, J. McManus & H. Köster Nucleic Acids Research 12 (11) 4539 (1984)), y compris la déprotection acide du groupe aminé terminal. Le clivage du produit du support solide est réalisé dans les conditions habituelles et le produit final est obtenu avec un rendement comparable à ceux couramment réalisés en synthèse oligonucléotidique.

EXEMPLE III -

Préparation de molécules mixtes oligodésoxyribonucléotide-macromolécule synthétiques ou naturelles utiles dans la détection de séquences d'ADN par des méthodes non-radioactives

La synthèse et l'utilisation de molécules mixtes composées d'une partie oligodésoxynucléotidique et d'une autre partie possédant des propriétés permettent la détection facile et rapide de cibles d'acides désoxyribonucléiques par des méthodes non-radioactives. La partie oligodésoxyribonucléotidique de séquence définie et homologue d'un fragment d'ADN cible apporte l'énergie de stabilisation qui assure son hybridation avec la molécule d'ADN que l'on veut détecter. La partie qui fournit la réactivité permettant la détection de l'hybride est couplée par un lien chimique covalent avec l'oligodésoxyribonucléotide. Parmi les différentes molécules possédant les propriétés requises pour la détection, on utilise de préférence la polybiotinylpolyéthylèneimine, la polyfluoresceinyl-polyéthylèneimine, la micropéroxydase, la phosphatase alkaline.

Le procédé de préparation de tels composés mixtes, présenté dans le schéma 3 ci-après, comprend soit quatre étapes, soit trois étapes principales, suivant la nature de la partie détectable de la molécule, désignée par l'"élément de marquage"M.

1) Synthèse de l'oligodésoxyribonucléotide en phase solide par une des différentes méthodes connues.

2) Extension en 5′ de l'oligonucléotide en phase solide par un nucléotide modifié chimiquement, porteur d'une chaîne (désignée par "bras") ayant à son extrémité un groupe fonctionnel ($R_1$) qui, après activation avec un réactif bifonctionnel, établit le lien covalent avec la molécule "M".

3) Greffage, le cas échéant, de groupes moléculaires détectables, sur un polymère, pour préparer M.

4) Couplage de l'oligodésoxyribonucléotide modifié avec la molécule "M".

La réaction de couplage 4) est effectuée avec la molécule oligonucléotidique, soit encore fixée sur le support solide de la synthèse, soit après clivage quand la stabilité de la molécule "M" est incompatible avec les conditions de clivage.

La synthèse oligonucléotidique peut être réalisée par la méthode au phosphite ou au phosphate manuellement ou dans un appareil automatique de synthèse comme décrit dans la littérature scientifique de la spécialité.

L'introduction du bras chimique nécessaire au couplage avec la molécule "M" se fait par l'intermédiaire d'un nucléotide modifié selon l'Exemple I qui peut être notamment la thymidine, la désoxyadénosine, la désoxycytidine, la désoxyguanosine ou la désoxyinosine protégées chimiquement sur la base, auquel on a fixé le "bras" dans son extrémité 5′ par une suite de réactions chimiques contrôlées, réalisées en solution. Ces réactions comprennent la pro tection sélective du groupe hydroxylique en 3′, l'activation du groupe hydroxylique en 5′ et la réaction avec un dinucléophile mono protégé qui crée en lien carbamate stable. Le dinucléophile est une diamine monoprotégée préparée nouvellement pour les besoins de la synthèse.

La fonction hydroxylique en 3′ du nucléoside est libérée de son groupe protecteur et phosphorylée avec des réactifs qui permettent le couplage en phase solide du nucléotide modifié, soit par la méthode du phosphate triester, soit par la méthode du phosphite avec un oligodésoxyribonucléotide chimiquement protégé.

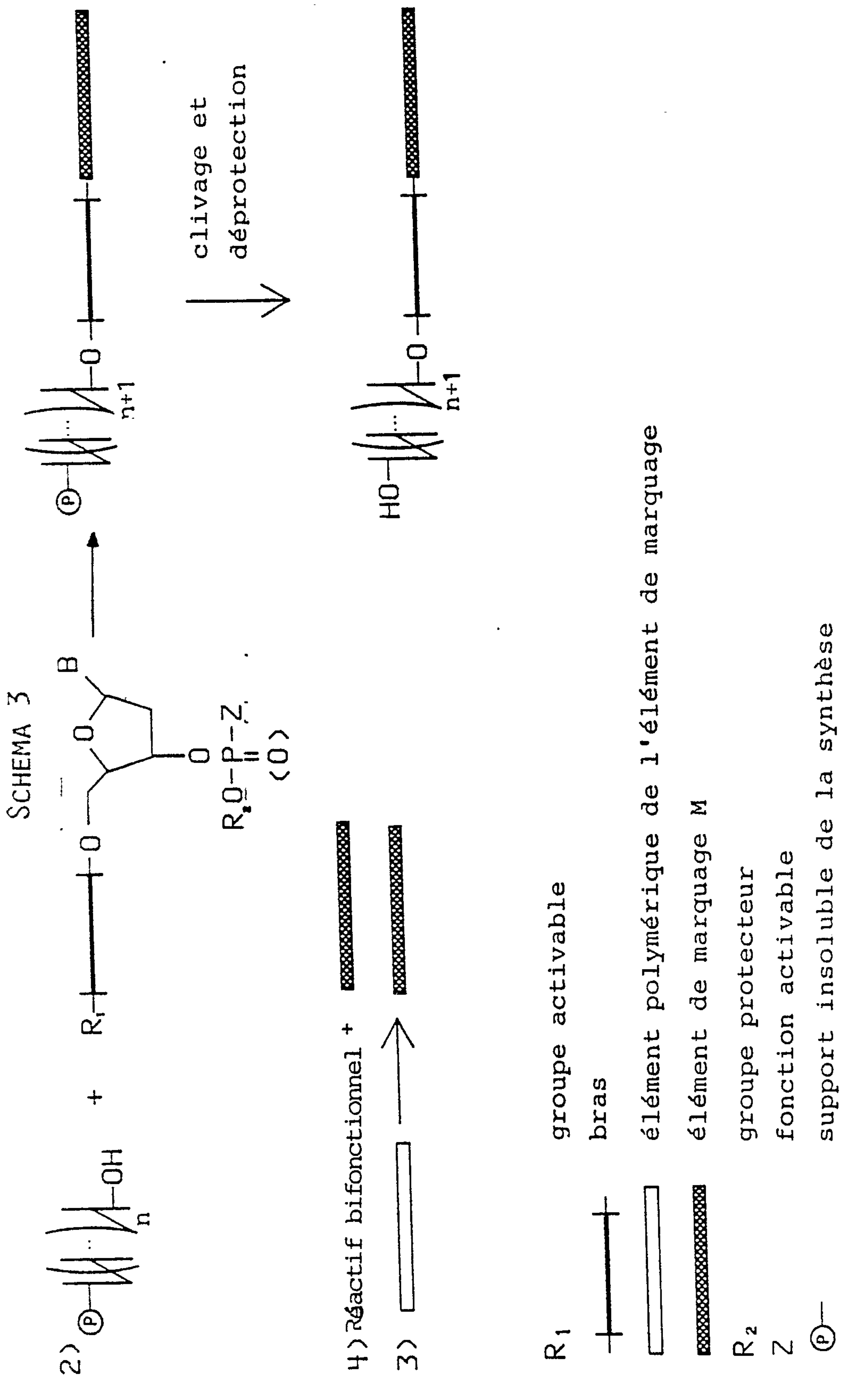


Le marquage des polymères chimiques se fait par l'intermédiaire des fonctions nucléophiles par réaction soit avec des esters activés dans le cas de la biotine ou avec le groupe isothiocyanate dans le cas de la fluorescéinéisothiocyanate. Dans des conditions de non saturation, il reste des groupes nucléophiles pour coupler le polymère marqué à l'oligonucléotide porteur du "bras" nucléophile. Ce couplage se fait donc par l'intermédiaire d'un réactif bifonctionnel tel qu'un diépoxyde, le diisocyanatohexane ou le diester

N-hydroxysuccinimidique de l'acide subérique.

1) Biotinylation de la polyéthylèneimine PG35

La fixation de la biotine sur la polyéthylèneimine a été étudiée en prenant comme modèle la N-hydroxysuccinimidoyl biotine tritiée. Après agitation des réactifs pendant plusieurs heures dans un milieu 0,1 M en $NaHCO_3$, on effectue la séparation entre les molécules polymériques et les molécules de biotine libres en deux étapes : dialyse dans un tampon phosphate puis filtration moléculaire. Le taux d'incorporation est mesuré par radioactivité. Les conditions mises au point sur ce modèle peuvent être reproduites avec un réactif plus élaboré tel que l'ester N-hydroxysuccinimidique de biotinoamidocaproate qui permet de soulager l'encombrement stérique entre les cycles biotinyliques et le squelette du polymère facilitant l'approche des complexes d'avidine à utiliser pour l'amplification et la détection.

Ces groupes biotinyles présentent une affinité très élevée pour l'avidine (ou streptavidine) et pour ses analogues conjugués (à la peroxydase ou à la phosphatase alcaline). Les complexes biotine-avidine conjugués peuvent alors être détectés par l'action de l'enzyme conjugué sur un substrat chromogénique comme le 5-bromo-4-chloro-3-indolyl-phosphate et le bleu de nitrotétrazolium.

La polyéthylèneimine a été biotinylée en agitant pendant 24 heures 700 µl d'une solution de PG35 à 10 % (BASF) - 50 µmoles avec 198 mg - 580 µmoles de N-hydroxysuccinimidoylbiotine dont une fraction des molécules est tritiée en position 8-9 (Amersham).

Le mélange réactionnel est ensuite dialysé contre une solution 5 mM $Na_2HPO_4$ (pH 8,2). On dialyse 3 fois 0,5 heures contre 40 cc de tampon phosphate. Le contenu du sac à dialyse est alors lyophylisé et le résidu est purifié par chromatographie sur colonne Sephadex G25 en éluant avec de l'$H_2O$.

Le taux d'incorporation mesuré par radioactivité indique une incorporation de 9,3 molécules de biotine par molécule de polymère ($\overline{Mw}$ 1400).

2) Procédé de préparation d'une sonde oligodésoxyribonucléotidique couplée à la polyéthylèneimine biotinylée.

Une sonde ADN a été synthétisée avec une séquence oligodésoxynucléotidique homologue d'une région spécifique de l'ADN d'un type du virus de papilloma humain (HPV) de 24 nucléotides.

On prépare, en phase solide dans un appareil automatique, une sonde de séquence 5′ TGG GCT CTG TCC GGT TCT GCT TGT 3′ dans laquelle le 24e nucléotide T est modifié comme décrit exemple 1. Pour ce faire, on a utilisé 6 mg de support CPG fonctionnalisé avec de la diméthoxytritylthymidine à 34 µmole/g, ce qui correspond à 0,2 µmole. Après assemblage automatique de l'icosatétramère (24-mer), la fonctionnalisation de la chaîne oligodésoxyribonucléotidique par le polymère biotinylé a été réalisée de trois manières différentes :

A. fonctionnalisation sur support solide par le polymère biotinylé ;

B. fonctionnalisation sur support solide par le polymère non biotinylé puis biotinylation du polymère fixé ;

C. fixation en solution, après clivage de la chaîne du support, du polymère biotinylé.

Méthode A : fonctionnalisation sur support solide par le polymère biotinylé.

Le 24-mer dont le 24e nucléotide T a été modifié (chaîne alkylaminée) est préparé au synthétiseur comme décrit ci-dessus. L'amine terminant la chaîne est déprotégée automatiquement par traitement à l'acide trichloroacétique, puis la colonne contenant l'oligomère fixé sur silice est enlevée de l'appareil et traitée de la façon suivante : la colonne est lavée, à la seringue, avec 2 cc d'acétonitrile sec puis séchée à l'argon et traitée pendant 2 h 30 avec 250 µl d'une solution 0,1 M en di N-hydroxysuccinimidoylsubérate dans le DMF sec. La colonne est ensuite lavée, à la seringue avec 2 cc de DMF sec puis 2 cc d'acétonitrile sec et séchée à l'argon. La colonne est alors traitée 14 heures avec 250 µl d'une solution 0,032 M en PG35,4 biotine dans un mélange 50/50 : $NaHCO_3$ 0,1 M - DMF. La colonne est ensuite lavée, à la seringue, avec 4 cc d'eau puis 2 cc d'acétonitrile et séchée à l'argon. La chaîne désoxyribonucléotidique ainsi fonctionnalisée est clivée du support CPG par 4 traitements successifs de 0,5 heures avec 500 µl de $NH_4OH$ 25 %. Les solutions ammoniacales réunies sont liophylisées, le résidu est dissous dans 3 cc de $NH_4OH$ 25 % et la solution est maintenue 5 heures à 50°C. Ce deuxième traitement ammoniacal a pour but de déprotéger les bases et les phosphates des chaînes oligonucléotidiques. La solution est alors liophylisée, le résidu est soumis à une chromatographie par filtration moléculaire (gel Sephadex G50) puis à une électrophorèse sur gel de polyacrylamide.

Alternativement, la purification finale est effectuée par HPLC sur une colonne de chromatographie d'exclusion (tampon phosphate pH 7,9, élution isocratique).

Méthode B : Fonctionnalisation sur support solide par le polymère non biotinylé suivi de la biotinylation du polymère fixé.

Le 24-mer dont le 24e nucléotide T a été modifié (chaîne alkylamine) est préparé au synthétiseur comme décrit ci-dessus. L'amine terminant la chaîne est déprotégée automatiquement par traitement à l'acide trichloroacétique, puis la colonne contenant l'oligomère fixé sur silice est enlevée de l'appareil et traitée de la façon suivante : la colonne est lavée, à la seringue, avec 2 cc d'acétonitrile sec puis séchée à l'argon et traitée pendant 2 h 30 avec 250 µl d'une solution 0,1 M en di N-hydroxysuccinimidoylsubérate dans le DMF sec. La colonne est ensuite lavée avec 2 cc de DMF sec, puis 2 cc d'acétonitrile sec et séchée à l'argon. Elle est

ensuite traitée pendant 19 heures avec 10 µmole de polyéthylèneimine PG35 (140 µl d'une solution à 10 % dilué avec 160 µl d'une solution 50/50 $NaHCO_3$ 0,1 M - DMF). La colonne est alors lavée avec 4 cc d'une solution aqueuse saturée en citrate de sodium puis 4 cc d'eau, 2 cc d'acétonitrile et séchée sur Argon. On traite alors la colonne 15 heures avec 250 µl d'une solution 0,05 M de N-hydroxysuccinimidoyl biotine tritiée. La colonne est alors lavée avec 4 cc d'eau, 2 cc d'acétonitrile et séchée à l'argon. La chaîne oligodésoxyribonucléotidique ainsi fonctionnalisée est clivée du support par 4 traitements successifs d'1/2 heure avec 500 µl de $NH_4OH$ 25 %. Les solutions ammoniacales sont réunies et liophylisées puis le résidu est dissous dans 3 cc de $NH_4OH$ 25 % ; la solution obtenue est maintenue 5 heures à 50°C. Ce deuxième traitement ammoniacal a pour but de déprotéger les bases et les phosphates des chaînes oligonucléotidiques. La solution est alors liophylisée ; le résidu est soumis à une chromatographie de filtration moléculaire (gel Sephadex G50), puis à une électrophorèse sur gel de polyacrylamide.

Alternativement, la purification finale est effectuée par HPLC sur une colonne de chromatographie d'exclusion (tampon phosphate pH 7,9, élution isocratique).

Méthode C : Fixation en solution, après clivage de la chaîne du support, du polymère biotinylé.

Le 24-mer dont le 24e nucléotide T a été modifié, est préparé au synthétiseur comme décrit ci-dessus. La déprotection à l'acide trichloroacétique de l'amine terminant la chaîne oligonucléotidique et le clivage de la chaîne du support à l'ammoniaque 25 % sont effectués automatiquement dans l'appareil. La solution ammoniacale obtenue, contenant la chaîne nucléotidique est portée à 3 cc par une solution $NH_4OH$ 25 % ; elle est traitée 5 heures à 50°C puis liophylisée. Le résidu est soumis à une chromatographie de filtration moléculaire (gel Sephadex G50) suivi d'une électrophorèse sur gel de polyacrylamide. L'oligodésoxyribonucléotide ainsi synthé tisé et purifié est soumis à une suite de traitements en solution en vue de la fixation du polymère PG35 biotinylé : 1,2 D.O. d'oligomère sont dissous dans 12 µl d'une solution aqueuse 0,1 M en $NaHCO_3$ et 2 mM en EDTA. On y ajoute 25 µl d'une solution de di N-hydroxysuccinimidoyl subérate dans le DMSO sec (10 mg/ml). On laisse le mélange réactionnel 10 minutes à température ambiante puis on purifie sur colonne Sephadex G25 éluant $H_2O$. La première fraction contenant de l'oligomère est refroidie à la sortie de la colonne et liophylisée. Après liophylisation, on ajoute au résidu 80 nmole de PG35,4 biotine dissous dans 100 µl d'une solution aqueuse 0,1 M $NaHCO_3$ et 3 M NaCl. Le mélange réactionnel est maintenu 20 heures à température ambiante, puis soumis à une chromatographie de filtration moléculaire.

Pour le couplage, l'oligonucléotide comportant le bras chimique aminé en son extrémité 5' peut être activé avec comme réactif bifonctionnel outre le di N-hydroxysuccinimidoyl subérate, le 1,2,7,8-diépoxyoctane ou le 1,6-diisocyanatohexane.

EXEMPLE IV - Emploi de la sonde oligodésoxyribonucléotidique liée à la polyéthylèneimine biotinylée à la détection des virus de papilloma humains

L'utilité de la molécule mixte oligodésoxyribonucléotide-macromolécule marquée à la biotine décrite plus haut a été exemplifiée dans des expériences d'hybridation et de détection dans lesquelles la molécule d'ADN cible est le génome du virus du papilloma humain 16. La molécule mixte préparée suivant l'une des méthodes décrite ci-dessus s'hybride spécifiquement avec l'ADN de HPV16 à l'exclusion des séquences d'ADN d'autres HPV. La détec tion des molécules hybrides a été effectuée au moyen des complexes biotine-streptavidine conjugués par l'action de l'enzyme conjuguée peroxydase ou phosphatase alkaline sur les substrats chromogéniques diaminobenzidine ou 5-bromo-4-chloro-3-indolyl-phosphate et bleu de nitrotétrazolium respectivement.

L'ADN de HPV16 et de HPV18 clonés dans des vecteurs plasmidiques a été déposé en parallèle sur des filtres de nylon ou sur des filtres de nitrocellulose. Ces filtres ont été préhybridés dans du 5X SSPE 5X Denhart, 0,5 % SDS, 0,2 µg/ml d'ADN de sperme de saumon, soit à 45°C, soit à 60°C. Les filtres ont été hybridés dans ce mélange d'hybridation contenant 0,1 µg/ml d'ADN de sperme de saumon et 200 ng de sonde HPV16 biotinylée soit à 45°C, soit à 60°C. Les filtres ont été lavés dans du 2X SSPE, 0,1 % SDS à température ambiante pendant 2 x 10' et 10' dans du 5X SSPE, 0,1 % SDS à 60°C ou 45°C. Plusieurs procédés différents ont été utilisés par la suite.

Procédé 1:

Les sites aspécifiques ont été bloqués pendant 1 h à 37°C dans le tampon A (tris HCL pH 7,5 0,1 M, NaCl 1 M, 2 mM $MgCl_2$, 0,5 % Tween 20, 0,05 % Triton X100) contenant 3 % de BSA. Les filtres ont été lavés 2 x dans ce tampon A, ensuite incubés dans le tampon B (Tris 0,1 M, NaCl 1M, $MgCl_2$ 2mM, 0,05 % Triton X100 et 0,05 % Tween 20) en présence d'un complexe streptavidine peroxidase biotinylée pendant 1 h à température ambiante. Les filtres ont ensuite été lavés 5 x dans le tampon A contenant 1 % BSA, puis 2 x dans le tampon C (Tris 20 mM pH 7,5 NaCl 0,5 M) et incubés pendant 30' au moins dans du tampon C en présence de diaminobenzidine et de $H_2O_2$.

Procédé 2 :

Les filtres ont été bloqués 20 minutes à 42°C dans le tampon 1 (Tris-HCl 0,1 M, pH 7,5 ; NaCl 0,1 M, 2 mM $MgCl_2$, 0,05 % Triton X-100 contenant 3 % BSA). Les filtres ont ensuite été séchés 20 minutes à 80°C sous vide, rehydratés dans le tampon 1 en présence de BSA. Les filtres ont été ensuite incubés 10 minutes dans le tampon 1 contenant 1 µg/ml de phosphatase alkaline de veau biotinylée. Après avoir été lavés dans le tampon

1, puis dans le tampon 3 (Tris-HCl 0,1 M pH 9,5 ; 0,1 M NaCl, 50 nM $MgCl_2$), les filtres ont été incubés un maximum de 4 heures à l'abri de la lumière dans du tampon 3 contenant du bleu de nitrotétrazolium et du 5-bromo-4-chloro-3 indolyl phosphate.

1 fentomole d'ADN de HPV16 peut être spécifiquement et facilement détectée par les deux méthodes avec les deux types de filtres, bien que les filtres en nylon présentent un bruit de fond élevé. L'ADN de HPV18 utilisé en parallèle comme contrôle négatif ne se colore point.

La figure 1 représente la visualisation de l'hybridation de l'ADN de HPV16 à une sonde ADN polybiotinylée comme décrit ci-dessus de HPV16.

L'ADN a été déposé dans des filtres de nitrocellulose.

La colonne 1 comportait de l'ADN de HPV16 cloné dans PBR 322.

La colonne 2 comportait de l'ADN de HPV18 cloné dans PBR 322.

Les différents filtres correspondaient pour

A à 5 µg d'ADN
B à 1 µg d'ADN
C à 0,1 µg d'ADN
D à 10 ng d'ADN
E à 1 ng d'ADN
F à 0,1 ng d'ADN.

L'hybridation s'est faite selon le procédé 2 dans le mélange de préhybridation en présence de 200 ng de sonde d'HPV16 biotinylée par ml de milieu. On observe une visualisation de l'hybridation pour 10 ng d'ADN de HPV16.

EXEMPLE V: Sonde marquée à la biotine directement avec détection après amplification de l'ADN à détecter

1) Synthèse automatique d'un oligonucléotide 29 mères spécifique de HPV16 et contenant en bout de chaîne de thymidine 5′ alkylaminée.

L'oligonucléotide de 29 mères spécifique de HPV16 synthétisé est le suivant :
5′ TAC GCA CAA CCG AAG CGT AGA GTC ACA CT 3′
alkylaminé

Il a été préparé par la méthode des phosphoramidites (McBride, L.J. et Caruthers, M.H., Tetrahedron Lett. 24 (1983) 245-248) sur un synthétiseur Applied Biosystems 380A. La synthèse a été menée à l'échelle de 0,2 µmole en utilisant des 2-cyanoéthyl N,N - diisopropyl phosphoramidites.

Le dernier phosphoramidite introduit sur la chaîne oligonucléotidique est le synthon dont la synthèse a été précédemment décrite :

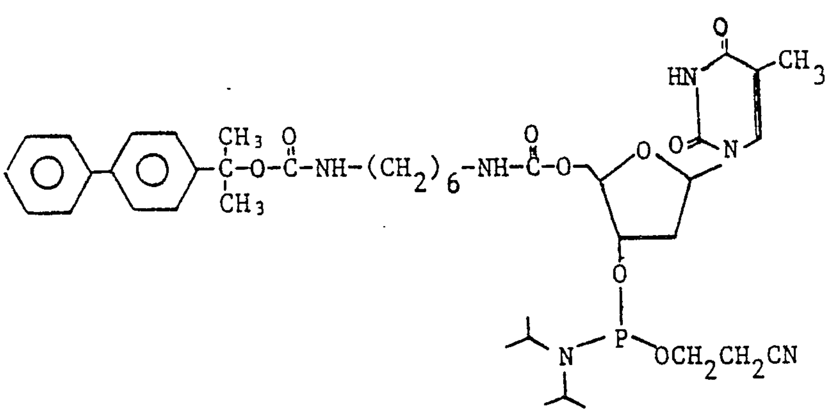

Après élimination (automatique) du groupe protecteur (le diphényl-isopropyloxycarbonyl) en bout de chaîne oligonucléotidique par l'acide trichloracétique à 3 % dans le dichlorométhane, le support "controlled porous glass" (CPG) portant la chaîne oligonucléotidique 5′ alkylaminée est traité comme décrit ci-dessous en vue d'un marquage à la biotine.

2) Marquage d'un oligonucléotide 5′ alkylaminé spécifique de HPV 16 par le sulfosuccinimidyl 6-(biotinamido)hexanoate.

L'emploi de ce dérivé de la biotine a pour effet d'allonger la distance avec l'oligonucléotide. Le nouveau lien ainsi créé entre le biotine et l'oligonucléotide est un groupe hydrocarboné en $C_{12}$. Le groupe sulfosuccinimidyl est un groupe partant substitué par l'amine terminal du bras en $C_6$ lié à l'oligonucléotide.

a) en phase solide

La moitié du support CPG portant la chaîne oligonucléotidique 5′ alkylaminée (0,1 µmole) (cfr préparation ci-dessus) est traitée 16 hrs par 250 µl d'une solution 0,1 M de sulfosuccinimidyl 6-(biotinamido)hexanoate (Pierce 21335) dans la diméthylformamide sèche (la diméthylformamide a été traitée une nuit sur oxyde de baryum puis filtrée et distillée sur hydroxyde de potassium, pression : 15 mm Hg).

Le support est ensuite lavé à l'eau (4 x 2cc) puis traité avec une solution ammoniacale à 25 % (4 x 250 µl - 1/4 hrs afin d'opérer le clivage de la chaîne oligonucléotidique du support. Les solutions ammoniacales réunies sont chauffées 5 heures à 55°C, pour déprotéger les amines primaires des bases de la chaîne oligonucléotidique, puis lyophillisées. Le résidu obtenu est désalé sur colonne SEP-PAK C18 (1° élution, 5cc eau ; 2° élution 5cc 20 % $CH_3CN$-80 % eau). On obtient 9,3 $DO_{257}$ de produit brut (échantillon 1 testé en hybridation, voir ci-après).

Six $DO_{257}$ du produit brut (échantillon 1) ont été purifiés par électrophorèse (gel de polyacrylamide 20 % - 1,5 mm). Le produit biotinylé est ensuite sorti du gel par électroélution (Tris 15 mM pH 8,3); la solution obtenue est lyophillisée et constitue l'échantillon 2 (0,375 $DO_{255}$), testé en hybridation ci-après.

Outre le produit biotinylé, on récupère encore, 1,02 $DO_{257}$ d'oligomère 5′ alkylaminé de départ qui constitue l'échantillon 3 testé en hybridation ci-après.

b) en phase liquide

La moitié du support CPG portant la chaîne oligonucléotidique 5′ alkylaminée (0,1 µmole) (cfr. préparation ci-dessus) est traitée à l'ammoniaque à 25 % (4 x 250 µl - 1/4 hrs) afin de cliver la chaîne oligonucléotidique du support. Les solutions ammoniacales réunies sont chauffées 5 hrs à 55°C, pour la déprotection des amines primaires des bases de la chaîne oligonucléotidique, puis lyophillisées. Le résidu obtenue est élué sur colonne (10 ml) Sephadex G50 (éluant TEAB 10 mM) puis purifié sur gel de polyacrylamide (20 % - 1,5 mm épaisseur). L'oligonucléotide est récupéré par électroélution (Tris 15 mM, pH 8,3). La solution d'oligonucléotide obtenue (5,4 $DO_{257}$) est lyophillisée.

Deux $DO_{257}$ d'oligonucléotide 5′ alkylaminé sont mis en solution dans 20 µl de tampon HEPES 0,2 M (pH 7,6). On ajoute à la solution 100 µl d'une solution 0,1 M de sulfosuccinimidyl 6-(biotinamido) hexanoate dans la diméthylformamide. Le mélange réactionnel est laissé 16 heures à température ambiante puis élué sur une colonne Sephadex G50 (10 ml ; éluant TEAB 100 mM). On récolte 1,96 $DO_{257}$ de produit brut pour lequel les tests en hybridation ont donné, tant au point de vue sélectivité que sensibilité, les mêmes résultats que l'échantillon 1 (brut de la biotinylation en phase solide).

3) Détection de l'ADN du virus du papillome 16 à l'aide de l'oligomère biotinylé.

a) Isolation des cellules de biopsies du col de l'utérus

Les biopsies de tumeurs sont dans un premier temps découpées au scalpel en morceaux d'environ 3 $mm^3$. Ces morceaux sont ensuite suspendus dans un tampon salin PBS (1X : NaCl 15 mM, $KH_2PO_4$ 2 mM) puis sont mis à digérer par la trypsine 0,25 % dans PBS. Les cellules désagglomérées sont ensuite comptées.

b) amplification enzymatique in vitro de l'ADN du virus du papillome (HPV)

L'ADN de différentes tumeurs a été engagé dans des réactions d'amplifications enzymatiques selon un procédé analogue à celui décrit par Saiki. (réf. 1). Trois paires d'amorces spécifiques chacunes d'un type d'HPV ont été utilisées dans cette expérience (réf. 2).

| Amorces spécifiques de l'HPV16 | position dans le génome d'HPV |
| --- | --- |
| GCA GAA CCG GAC AGA GCC CA | 694-713 |
| GTG TGC CCA TTA ACA GGT CTT CC | 820-798 |

Ces amorces définissent un fragment de 127 bp.

| Amorces spécifiques de l'HPV18 | |
| --- | --- |
| GCCCGACGAGCC-GAACCACA | 740-759 |
| GGAATGCTC-GAAGGTCGTCTG | 848-828 |

Ces amorces définissent un fragment de 109 bp.

| Amorces spécifiques de l'HPV33 | |
| --- | --- |
| GGCTTGGACCGGC-CAGATGG | 681-700 |
| GTGCACAGGTAGGG-CACACAA | 858-839 |

Ces amorces définissent un fragment de 178 bp.

La réaction d'amplification se fait dans un volume de 50 µl de mélange contenant six mille cellules de tumeurs, 16,6 mM $(NH_4)_2SO_4$, 67 mM Tris-HCl pH 8,8, 6,7 mM $MgCl_2$, 10 mM BME, 200 µm dATP, dCTP, dGTP, dTTP, 200 µg/ml de gélatine et 30 picomoles de chacune des amorces, 0,25 % DMSO, 1 unité de polymérase thermostable (Taq polymérase, New England Biolabs). Une goutte d'huile minérale est ajoutée et le mélange est incubé 10 minutes à 92° C. Ensuite, il est incubé successivement 1 minute à 92° C, 3 minutes à 66° C et le cycle est répété trente fois.

c) Préparation des filtres pour l'hybridation avec l'oligomère biotinylé

D'une part, l'ADN cloné dans le pBR322 des HPV6b, 11, 16, 18 et 33 a été dénaturé 15′ à 45° C dans du NaOH 0,5 N. Chaque clone a été filtré sur une membrane de nylon, en présence d'1 µg d'ADN carrier, l'ADN de sperme de saumon. Les filtres sont placés 10 minutes sur un coussin imbibé de NaOH 0,5 M, NaCl 1,5 M, 10 minutes sur un coussin imbibé de 0,5 M Tris-HCL pH 7,5 NaCl 1,5 M et rincés 5 minutes dans du 2XSSC (1XSSC = 0,15 M NaCl, 15 mM citrate trisodique pH 7,2). Les filtres sont alors séchés à l'air, puis l'ADN est fixé par irradiation aux UV (312 nm, pendant 1 minute).

D'autre part, un cinquième du produit de chaque amplification enzymatique est analysé par électrophorèse en gel d'agarose Nusieve 4 %. Le gel est alors incubé 30 minutes dans une solution de NaOH 0,5 M, NaCl 1,5 M (dénaturation de l'ADN) puis il est neutralisé par une incubation de 30 minutes dans du Tris-HCl pH 7,5, NaCl 1,5 M. L'ADN est transféré sur membrane de nylon par la technique de Southern (réf. 3) et fixé par irradiation aux U.V.

d) Hybridation des filtres

Les filtres sont préhybridés dans le mélange suivant : 5XSSPE (1XSSPE = 0,18 M NaCl, $NaH_2PO_4$ 10 mM, EDTA pH 8 1 mM), 5X Denhart (1X Denhart = 0,02 % Ficoll, 0,02 % BSA, 0,02 % polyvinylpyrrolidone), 0,1 mg/ml d'ADN de sperme de saumon dénaturé à 45° C pendant un minimum de deux heures.

On rajoute alors l'échantillon 1,2 ou 3 dilué (1 µg/ml) dans le mélange de préhybridation et l'hybridation se fait à 45° C pendant 16 heures.

Les filtres sont ensuite lavés deux fois 15 minutes dans du 3XSSC, 0,1 % SDS à température ambiante et 10 minutes dans du 6XSSC, 0,1 % SDS à 45° C.

e) Détection des hybrides biotinylés

Les filtres sont incubés 20 minutes dans une solution bloquante à 42° C (0,1 M Tris-HCl pH 7,5, 0,1 M NaCl, 2 mM $MgCl_2$, 0,05 % Triton X-100 = tampon 1 + 3 % BSA = tampon 2). Les filtres sont ensuite séchés puis réhydratés dans le tampon 2, puis incubés 10 minutes en présence de streptavidine (2 µg/ml de tampon 1). Les filtres sont rincés trois fois dans le tampon 1, puis incubés 10 minutes en présence de biotine couplée à la

phosphatase alcaline (1 µg/ml tampon 1). Les filtres sont rincés deux fois dans le tampon 1 puis deux fois dans le tampon 3 (0,1 M Tris-HCl pH 9,5, 0,1 M NaCl, 50 mM $MgCl_2$).

Les filtres sont alors incubés dans le tampon 3 en présence du substrat de la phosphatase alcaline, le 5-bromo-4-chloro-3-indolyl phosphate (BCIP) et le bleu de nitrotétrazolium (NBT). La réaction de coloration est arrêtée par un lavage dans une solution de 20 mM Tris-HCl pH 7,5, 5mM EDTA.

f) Résultats

Nous avons dans un premier temps testé la sensibilité de la détection de l'échantillon 1 sur des quantités décroissantes ($10^{-13}$ à $10^{-16}$ moles: 100,10, 1 et 0,1 fentomoles) d'ADN de clone d'HPV6b, HPV11, HPV16, HPV18 et HPV33.

Nous avons pu remarquer que l'échantillon 1 est exclusivement spécifique de l'HPV16 et que le seuil de détection avoisinait la fentomole ($10^{-15}$).

Puis nous avons testé cet échantillon sur l'ADN de tumeurs amplifié enzymatiquement. Nous avons pu aisément, après une demi-heure de révélation, détecter des bandes spécifiques à l'HPV16.

Nous avons ensuite testé les échantillons 2 et 3 sur de l'ADN cloné. L'échantillon 2 a un seuil de détection abaissé d'au moins un facteur 10 ($10^{-16}$ mole) et conserve la spécificité à l'HPV16. L'hybridation avec l'échantillon 3 ne donne aucun signal visible, ce qui est attendu puisqu'il n'est pas biotinylé.

## REFERENCES


1. Saiki, R.K. et al. (1988) Science 239, 487-491.
2. Sondes d'acides nucléiques de virus de papillome humain. Herzog et al., 8710917.
3. Southern, E.M (1977) J. Mol. Biol. 98, 503.


## Revendications

1. Sonde d'acides nucléiques comportant une séquence d'acides nucléiques, caractérisée en ce que ladite séquence est liée à son extrémité 5', par l'intermédiaire d'un "bras" chimique difonctionnel divalent L, à un "élément de marquage" M, M étant une molécule synthétique ou naturelle, directement ou indirectement détectable de manière non isotopique selon la formule I :

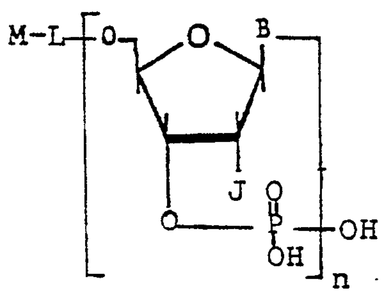

I

dans laquelle
J = H ou OH
n représente le nombre de nucléotides de 1 à 100.000
B = une base d'acides nucléique, purique ou pyrimidique, variable selon les nucléotides, le cas échéant.

2. Sonde selon la revendication 1, caractérisée en ce que L représente un reste chimique pouvant créer un lien carbamate avec le groupe hydroxylique 5' de la séquence d'acides nucléiques.

3. Sonde selon la revendication 2, caractérisée en ce que L représente un reste de formule III :

$$-NH-Alk-NH-\underset{\underset{O}{\|}}{C}- \quad III$$

dans laquelle
Alk représente une chaîne Alkyle droite ou ramifiée de 2 à 20 atomes de carbone.

4. Sonde selon la revendication 3, caractérisée en ce que L représente un reste de formule III dans laquelle Alk représente une chaîne droite de 2 à 12 carbones.

5. Sonde selon la revendication 4, caractérisée en ce que Alk représente le groupe $-(CH_2)_6-$.

6. Sonde selon l'une des revendications précédentes, caractérisée en ce que M est choisi parmi :
- des molécules directement détectables comme
. des enzymes appropriées à la révélation par un substrat chromogénique, telles que la microperoxydase et la phosphatase alcaline,
. des macromolécules synthétiques ou naturelles sur lesquelles sont fixées plusieurs molécules directement détectables, comme la fluorescéine.
- des molécules indirectement détectables comme

. des anticorps, la biotine, ou un de ses dérivés avec une chaîne hydrocarbonée,
. et des macromolécules synthétiques ou naturelles sur lesquelles sont fixées plusieurs molécules indirectement détectables telles que la biotine.

7. Composé utile notamment comme "élément de marquage" M selon la revendication 6, caractérisé en ce qu'il est choisi parmi des polymères synthétiques sur lesquelles sont fixées plusieurs molécules directement ou indirectement détectables comme
- le polybiotinylpolyéthylèneimine et
- le polyfluorescéinylpolythylèneimine.

8. Procédé de préparation de sondes selon l'une des revendications précédentes comportant :
a) la synthèse d'une séquence d'acides nucléiques, de préférence par toute méthode de synthèse d'assemblage inter-nucléotidique manuelle ou automatique connue, de préférence encore sur support solide, caractérisé en ce que ladite séquence est soumise, par une même méthode de synthèse, de préférence sur support solide, à
b) une extension en son extrémité 5′(OH) libre, sur l'extrémité 3′(OH) d'un synthon nucléotidique ou désoxynucléotidique, ce dernier étant protégé en 3′ par un groupe phosphorylé approprié au type de synthèse employé et, étant porteur en 5′ du "bras" chimique L protégé à son extrémité libre par le groupe protecteur $R_1$, lequel, après activation, établit
c) le couplage avec la molécule, "élément de marquage" M.

9. Procédé selon la revendication 8, caractérisé en ce que la synthèse est effectuée sur support solide, le couplage de l'étape c) est effectué quand le substrat produit des étapes a) et b) est encore fixé sur le support solide ou bien après clivage dudit substrat et du support solide.

10. Procédé selon l'une des revendications 8 et 9, caractérisé en ce que lorsque M représente une macromolécule sur laquelle sont fixés un ou plusieurs groupements directement ou indirectement détectables, la fixation desdits groupements sur ladite macromolécule a lieu avant ou après le couplage de cette dernière sur le substrat.

11. Composé intermédiaire utile notamment pour le procédé selon l'une des revendications 8 à 10, caractérisé en ce qu'il répond à la formule générale IV:

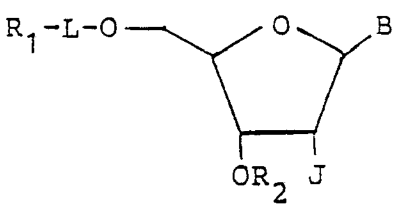

IV

dans laquelle
- J, B, L, $R_1$ ont les significations données précédemment, B étant éventuellement protégée,
- $R_2$ représente H ou tout groupe phosphorylé, éventuellement protégé, approprié à l'introduction du composé de formule IV à l'extrémité 5′ d'un autre nucléotide pour un type donné de synthèse d'assemblage inter-nucléotidique.

12. Composé selon la revendication 11, utile notamment pour le procédé selon la revendication 8 en cas de synthèse au phosphoramidite sur support solide, caractérisé en ce que $R_2$ représente le groupe cyanoéthyldiisopropylphosphoramidite de formule V

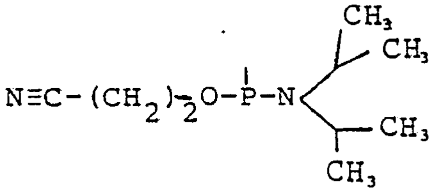

V

13. Composé selon l'une des revendications 11 et 12, caractérisé en ce que L représente un groupe de formule $-L'-\underset{\underset{O}{\|}}{C}-$

où L′ est un reste diaminé, le groupe protecteur $R_1$ de la fonction amine terminale de L′ est le groupe diphénylisopropyloxycarbonyle, labile en con ditions acides, de formule VI

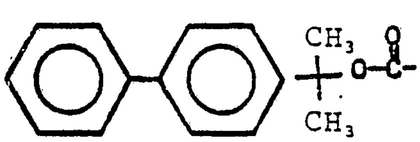

VI

14. Composé selon la revendication 13, caractérisé en ce qu'il répond à la formule VII

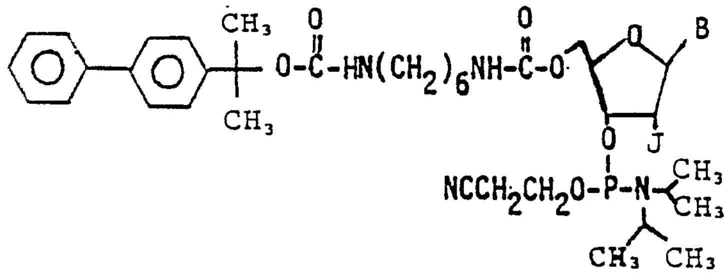

VII

dans laquelle B et J ont les significations données précédemment.

15. Procédé de préparation du produit selon l'une des revendications 11 à 14, caractérisé en ce qu'il comporte les étapes suivantes :

1) la protection sélective du groupe hydroxylique en 3′ du nucléoside de formule VIII

VIII

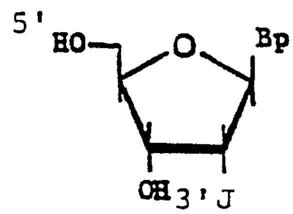

dans laquelle Bp est une base protégée et J = H ou OH.

2) L'activation sélective du groupe hydroxylique en 5′.

3) Une réaction de couplage en 5′ pour donner le "bras" chimique monoprotégé de formule $R_1$-L, ou $R_1$ et L ont les significations données précédemment.

4) La déprotection de la fonction hydroxylique en 3′ et éventuellement,

5) la phosphorylation avec un réactif qui permet le couplage en phase solide du nucléotide ainsi modifié en 3′, couplage à l'extrémité 5′ d'un autre nucléotide par un type donné de synthèse d'assemblage internucléotidique.

16. Procédé selon la revendication 15, caractérisé en ce que lorsque le bras L peut être représenté par la formule $L'-\underset{\underset{O}{\|}}{C}-$

dans laquelle L′ représente un reste diaminé, l'activation sélective du 5′OH se fait de préférence avec le groupe carbonyle diimidazole (CDI), la réaction de couplage de l'étape 3) se faisant avec le reste diaminé monoprotégé sur une amine terminale de formule $R_1$-L′, $R_1$ représentant de préférence le groupe diphénylisopropyloxycarbonyle.

17. Procédé selon l'une des revendications 15 ou 16, caractérisé en ce que la protection sélective 1) du groupe hydroxylique en 3′ se fera de préférence avec le groupe diméthyltertiobutylsilyle.

18. Procédé selon la revendication 17, caractérisé en ce que la déprotection de l'étape 4) se fait avec le fluorure de tétrabutylammonium.

19. Procédé selon l'une des revendications 17 et 18, caractérisé en ce que dans l'étape 5) la phosphorilation éventuelle en cas de synthèse au phosphoramidite se fait avec le diisopropylaminocyanoéthoxychlorophosphine.

20. Procédé selon l'une des revendications 15 à 19, caractérisé en ce que l'étape 1) se détaille de la manière suivante :

1a) - Protection sélective de l'extrémité 5′OH du nucléoside de formule VIII, par un groupe labile à pH acide, de préférence cette protection se faisant avec le diméthoxytrityle (DMT).

1b) - Protection du 3′OH du composé de formule VIII par un groupe labile dans des conditions douces et neutres, de préférence, cette protection se faisant par le groupe diméthyltertiobutylsilyle.

1c) - Déprotection sélective du DMT pour libérer le 5′OH.

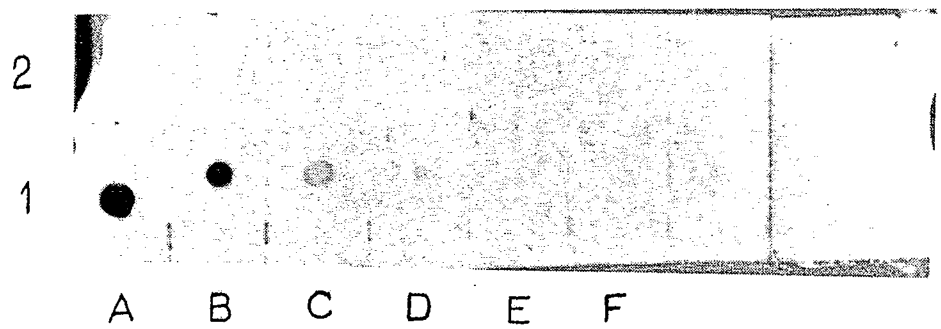
2
1
A B C D E F

Office européen des brevets

# RAPPORT DE RECHERCHE EUROPEENNE

Numero de la demande

EP 89 40 0494

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | WO-A-8 605 518 (J. SUMMERTON et al.) * Page 6, ligne 29 - page 8, ligne 17; page 79, exemple 13; page 89, exemple 20; page 90, exemple 21; page 93, exemple 23; page 94, exemple 24; page 95, exemple 25; page 96, exemple 26; page 98, ligne 3 - page 105, ligne 34 * | 1-6,8-16 | C 12 Q 1/68 |
| X | EP-A-0 230 363 (CETUS CORP.) * Page 2, ligne 3 - page 3, ligne 8; page 5, lignes 1-32; page 6, ligne 14 - page 7, ligne 29; page 8, ligne 7 - page 9, ligne 16; page 10, ligne 22 - page 14, ligne 12; page 41, ligne 2 - page 46, ligne 8 * | 1-6,8-16 | |
| X | EP-A-0 131 830 (MOLECULAR DIAGNOSTICS INC.) * Page 1, ligne 3 - page 9, ligne 35; page 11, lignes 22-32; page 13, lignes 23-36; page 22, ligne 2 - page 23, ligne 20 * | 1,6,9 | **DOMAINES TECHNIQUES RECHERCHES (Int. Cl.4)** |
| X | EP-A-0 244 860 (ENZO BIOCHEM, INC.) * Colonne 10, ligne 52 - colonne 12, ligne 36; colonne 14, ligne 28 - colonne 21, ligne 25 * | 1-5 | C 12 Q<br>C 12 N<br>C 07 H<br>G 01 N |
| X | EP-A-0 173 251 (BOEHRINGER MANNHEIM GmbH) * Page 2, ligne 27 - page 3, ligne 23; page 14, ligne 1 - page 17, ligne 30 * | 1,3-6,8,9,13-15 | |

-/-

Le présent rapport a été établi pour toutes les revendications

| Lieu de la recherche | Date d'achèvement de la recherche | Examinateur |
|---|---|---|
| LA HAYE | 25-05-1989 | VAN BOHEMEN C.G. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

Office européen des brevets

# RAPPORT DE RECHERCHE EUROPEENNE

Numero de la demande

EP 89 40 0494

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | DNA, vol. 4, no. 1, 1985, page 93, US; J.L. RUTH et al.: "Linker arm nucleotide analogs useful in oligonucleotide synthesis"<br>* En entier *<br>--- | 1,3-6, 11,13-16 | |
| X | NUCLEIC ACIDS RESEARCH, vol. 14, no. 15, 1986, pages 6227-6245, Oxford, GB; S. AGARWAL et al.: "Efficient methods for attaching non-radioactive labels to the 5' ends of synthetic oligodeoxyribonucleotides"<br>* En entier *<br>----- | 1-20 | |

DOMAINES TECHNIQUES RECHERCHES (Int. Cl.4)

Le présent rapport a été établi pour toutes les revendications

| Lieu de la recherche | Date d'achèvement de la recherche | Examinateur |
|---|---|---|
| LA HAYE | 25-05-1989 | VAN BOHEMEN C.G. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)